# EUROPEAN PATENT APPLICATION

(11) **EP 4 691 516 A1**
(43) Date of publication of application: **11.02.2026**
(21) Application number: 24823110.2
(22) Date of filing: 23.04.2024
(51) Int. Cl.: A61M 5/142

(54) **MEDICAL DEVICE**

(30) Priority: 12.06.2023 JP 2023096172
(71) Applicant: Terumo Kabushiki Kaisha, Tokyo 151-0072 (JP)
(72) Inventor: NAKAMOTO, Masato, Ashigarakami-gun, Kanagawa 259-0151 (JP); ARAKI, Takahito, Tokyo 163-1450 (JP); HASEGAWA, Makoto, Ashigarakami-gun, Kanagawa 259-0151 (JP)
(74) Representative: Prüfer & Partner mbB Patentanwälte · Rechtsanwälte
(86) International application number: PCT/JP2024/015877
(87) International publication number: WO 2024/257488

(57) **Abstract**

A medical device (10) includes a housing (16), an adhesion member (20), and an attachment (18). The adhesion member includes an adhesion body (106) fixed to a first outer surface (61) of the housing, and an overhanging portion (107) that extends from the adhesion body so as to overhang outward from the housing and is pressed by the attachment in an attached state of the attachment to be deformed along a second outer surface (69) of the housing, the overhanging portion includes an overhanging body (110), and an easily deformable portion (112) having lower rigidity than that of the overhanging body, and the easily deformable portion is provided in at least a portion deformed between the attachment and the housing in the attached state of the overhanging portion.

## Description

### Technical Field

The present invention relates to a medical device.

### Background Art

For example, Japanese Patent Application Laid-Open No. 2013-63105 discloses a liquid medicine administration device (medical device) including a housing and a flexible sheet-shaped adhesion member that is provided in the housing and can be adhered to the skin of a living body.

### Summary of Invention

There is a demand for a medical device capable of stably adhering an adhesion member to the skin of a living body.

An object of the present invention is to solve the above-described problem.
(1) An aspect of the present invention is a medical device including a housing, and a flexible sheet-shaped adhesion member that is provided in the housing and includes an adhesion surface capable of being adhered to a skin of a living body, the medical device including an attachment that is detachably attached to the housing, in which the adhesion member includes an adhesion body that is fixed to a first outer surface of the housing, and an overhanging portion that extends from the adhesion body so as to overhang outward from the housing, and is pushed by the attachment in an attached state in which the attachment is attached to the housing and deforms along a second outer surface adjacent to the first outer surface of the housing, the overhanging portion includes an overhanging body, and an easily deformable portion having lower rigidity than rigidity of the overhanging body, and the easily deformable portion is provided at least a portion deformed (for example, curved to be deformed) between the attachment and the housing in the attached state of the overhanging portion.
   According to such a configuration, since the overhanging portion is deformed along the second outer surface of the housing in the attached state of the attachment, the medical device can be made compact. Since the easily deformable portion is provided in the deformed portion of the overhanging portion, the overhanging portion easily returns to its original shape when the attachment is detached from the housing. That is, it is possible to prevent the deformation from remaining so that the overhanging portion rises to the side opposite to the direction in which the adhesion surface faces with respect to the adhesion body when the attachment is detached from the housing. As a result, the entire adhesion surface can be held in a planar shape when the attachment is detached from the housing. Since the overhanging portion includes the overhanging body having higher rigidity than that of the easily deformable portion, the overhanging portion can be easily pressed with fingers as compared with a case where the entire overhanging portion is formed of the easily deformable portion. Therefore, the adhesion member can be stably adhered to the skin of the living body.
(2) The medical device according to the above-described item (1), in which the adhesion member may include an adhesion sheet including the adhesion surface, and a base sheet provided on a surface on a side opposite to the adhesion surface of the adhesion sheet to support the adhesion sheet. According to such a configuration, it is easy to set the adhesion member to have appropriate rigidity by the base sheet.
(3) The medical device according to the above-described item (2), in which the overhanging body may include at least a part of the base sheet, and the easily deformable portion does not necessarily include the base sheet. According to such a configuration, the easily deformable portion can be easily provided in the overhanging portion.
(4) The medical device according to the above-described item (2), in which each of the overhanging body and the easily deformable portion may include at least a part of the base sheet, the base sheet may include a first portion that forms the easily deformable portion, and a second portion that forms the overhanging body, and the first portion may be thinner than the second portion. According to such a configuration, the easily deformable portion can be easily provided in the overhanging portion.
(5) The medical device according to the above-described item (4), in which the base sheet may be provided with a groove, and the first portion may be formed of the groove. According to such a configuration, the rigidity of the easily deformable portion can be easily reduced.
(6) The medical device according to the above-described item (5), in which the groove may be formed on a surface facing the adhesion sheet of the base sheet. According to such a configuration, when the attachment is attached to the housing, the overhanging portion can be easily bent to the side opposite to the adhesion surface. Therefore, the attachment can be easily attached to the housing.
(7) The medical device according to any one of the above-described items (2) to (6), in which the adhesion body does not necessarily include the base sheet. According to such a configuration, the configuration of the adhesion member can be simplified.
(8) The medical device according to any one of the above-described items (1) to (7), in which the housing may extend in one direction, the overhanging portion may extend outward in a width direction intersecting an extending direction of the housing from the adhesion body, and the easily deformable portion may extend over an entire length of the adhesion member in the extending direction of the housing. According to such a configuration, a dimension of the medical device in the width direction of the housing can be made compact in a state in which the attachment is attached to the housing.
(9) The medical device according to any one of the above-described items (1) to (8), in which a release sheet may be provided on the adhesion surface so as to be releasable, a tab may be provided on an outer periphery of the release sheet, and the tab is not necessarily provided with the adhesion member. According to such a configuration, the release sheet can be released from the adhesion surface in a state in which the tab is pinched.
(10) The medical device according to the above-described item (9), in which the tab may be a first tab, a second tab may be provided on an outer periphery of the adhesion member, and the second tab is not necessarily provided with the adhesion surface. According to such a configuration, the adhesion surface can be released from the skin in a state in which the second tab is pinched.
(11) The medical device according to the above-described item (10), in which the first tab and the second tab may be located so as to overlap each other and separated from each other as viewed in a thickness direction of the adhesion member. According to such a configuration, since the first tab and the second tab can be simultaneously processed, the number of manufacturing steps of the medical device can be reduced. The configuration of the medical device is simplified. Furthermore, since the first tab and the second tab are separated from each other, only the first tab can be easily pinched when the release sheet is released from the adhesion surface.
(12) The medical device according to any one of the items (1) to (11), in which a release sheet may be releasably provided on the adhesion surface, and the release sheet may be one continuous sheet. According to such a configuration, a releasing operation of the release sheet can be efficiently performed.
(13) The medical device according to any one of the above-described items (1) to (11), in which a release sheet may be releasably provided on the adhesion surface, an opening penetrating the adhesion member and the release sheet may be formed in the adhesion member and the release sheet in a thickness direction of the adhesion member, the release sheet may be divided into a plurality of divided sheets by a division line, and the division line may be provided at a position away from the opening so as not to be connected to an outer periphery of the opening. According to such a configuration, it is possible to suppress the release sheet from being broken when the release sheet is released from the adhesion surface.
(14) The medical device according to any one of the above-described items (1) to (13), in which a prefilled syringe and a puncture needle for administering a liquid medicine accommodated in the prefilled syringe to a living body may be provided inside the housing, in the housing, a needle leadout port for exposing a needle tip of the puncture needle may be formed, and the attachment may seal the needle leadout port in a state of being attached to the housing. According to such a configuration, it is possible to obtain the medical device for administering the liquid medicine to the living body.

According to the present invention, the adhesion member can be stably adhered to the skin of the living body.

### Brief Description of Drawings

Fig. 1 is a perspective view of a medical device according to an embodiment of the present invention.
Fig. 2 is an exploded perspective view of the medical device.
Fig. 3 is an exploded perspective view of the medical device.
Fig. 4 is a plan view of an adhesion sheet.
Fig. 5 is a cross-sectional view taken along a line V-V in Fig. 4.
Fig. 6 is a cross-sectional view taken along a line VI-VI in Fig. 5.
Fig. 7 is an illustrative diagram of use of the medical device.
Fig. 8 is an illustrative diagram of use of the medical device.
Fig. 9 is an illustrative diagram of use of the medical device.
Fig. 10 is an illustrative diagram of an adhesion member according to a first modification.
Fig. 11 is an illustrative diagram of an adhesion member according to a second modification.
Fig. 12 is an illustrative diagram of an adhesion member according to a third modification.
Fig. 13 is an illustrative diagram of a release sheet according to a modification.

### Description of Embodiments

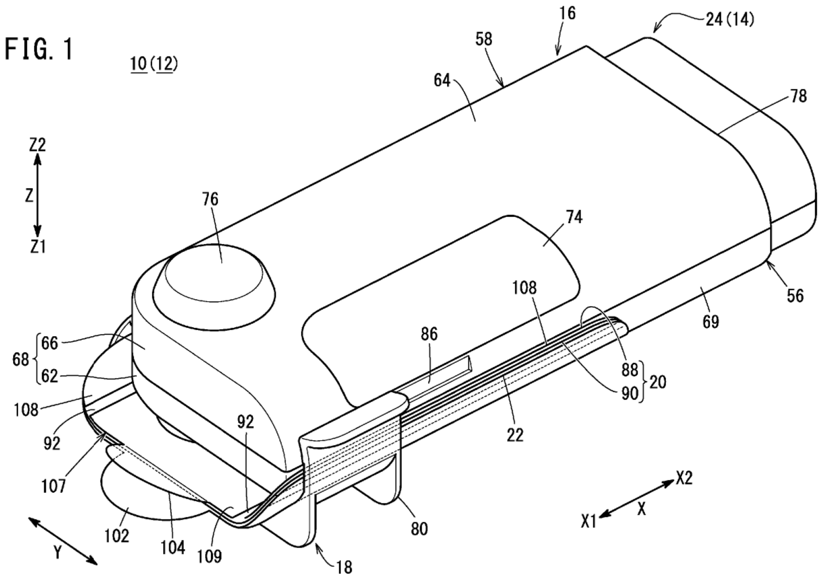

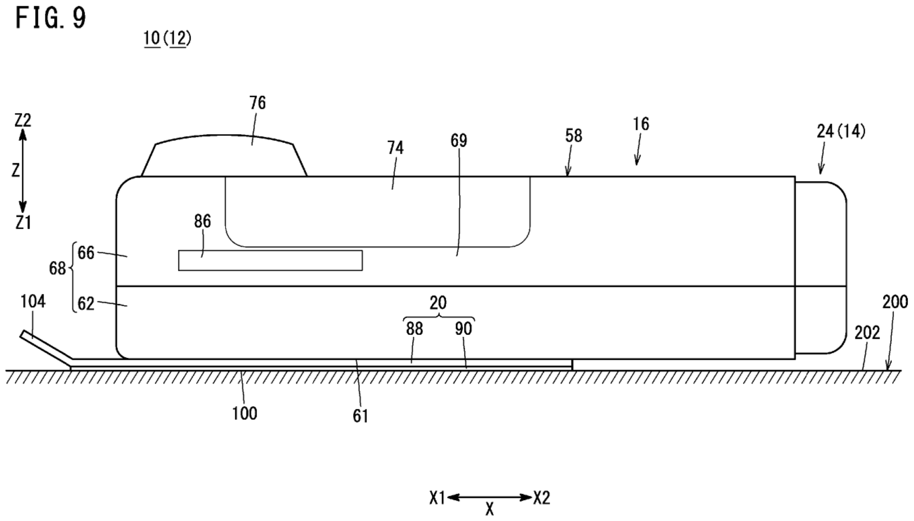

A medical device 10 according to one embodiment of the present invention will be described below with reference to the drawings. As illustrated in Figs. 1 and 9, the medical device 10 according to one embodiment of the present invention is a patch-type liquid medicine administration device 12 to be adhered to a skin 202 of a patient being a living body 200.

The liquid medicine administration device 12 continuously administers a liquid medicine into the living body 200 over a relatively long time (for example, about several minutes to several hours). The liquid medicine administration device 12 may intermittently administer the liquid medicine into the living body 200. Examples of the liquid medicine include protein preparations such as insulin preparations, narcotic analgesics, diuretics and the like.

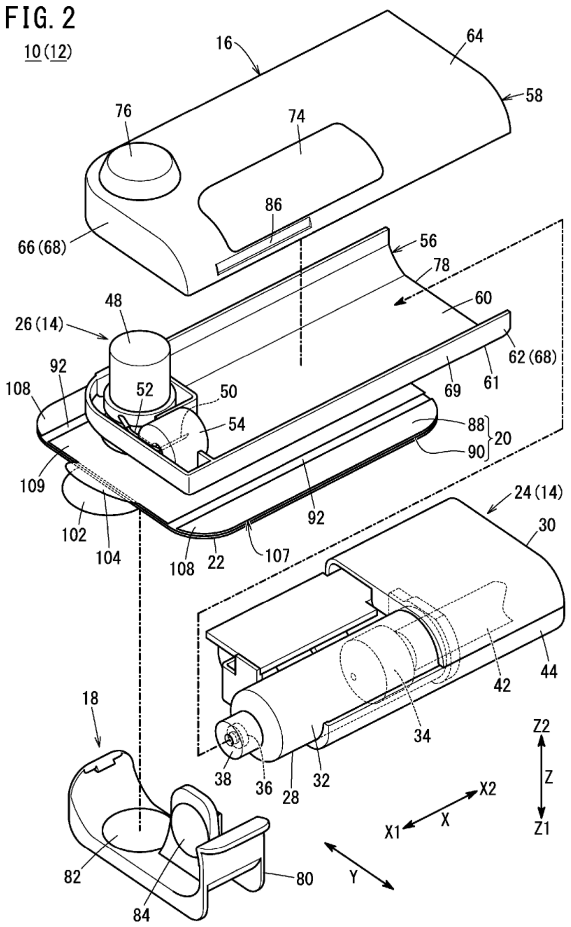

As illustrated in Fig. 2, the liquid medicine administration device 12 includes a device body 14, a housing 16, an attachment 18, an adhesion member 20, and a release sheet 22.

The device body 14 includes a syringe pump 24 and a puncture unit 26. The syringe pump 24 includes a prefilled syringe 28 and a drive unit 30. The syringe pump 24 is disposed inside the housing 16 in a slidable state in a longitudinal direction of the housing 16 (X direction).

The prefilled syringe 28 includes a syringe body 32, a gasket 34, a sealing member 36, and a distal end cap 38. The syringe body 32 is formed in a hollow cylindrical shape including a liquid medicine chamber inside. The liquid medicine is filled in the syringe body 32 in advance. The syringe body 32 is preferably formed of a material having transparency. A proximal end (end in an X2 direction) of the syringe body 32 is opened.

The gasket 34 is slidably disposed inside the syringe body 32. The gasket 34 is formed of, for example, an elastic resin material such as a rubber material and an elastomer material. An outer peripheral surface of the gasket 34 is in close contact with an inner peripheral surface of the syringe body 32 in a liquid-tight manner.

The sealing member 36 liquid-tightly seals a distal end opening of the syringe body 32. The sealing member 36 is formed of an elastic resin material such as a rubber material and an elastomer material. The distal end cap 38 prevents the sealing member 36 from coming out of the syringe body 32. The distal end cap 38 is formed of, for example, a hard resin material.

The drive unit 30 supports the prefilled syringe 28. The drive unit 30 includes a pusher 42 for pressing the gasket 34 in the distal end direction, and a drive cover unit 44 that supports the prefilled syringe 28. The drive unit 30 includes a power supply unit (for example, a battery), a motor, a power transmission mechanism, a control unit and the like.

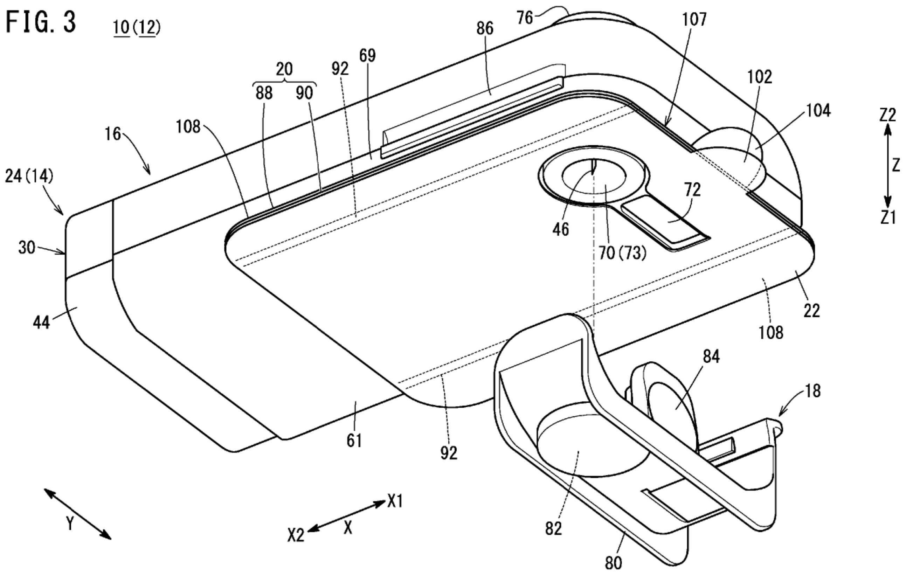

The puncture unit 26 is attached to the housing 16. The puncture unit 26 includes a puncture needle 46 (refer to Fig. 3), a needle support 48, a connection needle 50, a needle connection portion 52, and a cylinder 54. As illustrated in Fig. 3, the puncture needle 46 includes a needle tip capable of puncturing the skin 202 of the living body 200. The puncture needle 46 is formed in a tubular shape. The puncture needle 46 extends in a thickness direction of the housing 16 (Z direction).

As illustrated in Fig. 2, the needle support 48 is disposed in the housing 16 to support the puncture needle 46. The connection needle 50 includes a needle tip capable of penetrating the sealing member 36. The connection needle 50 is formed in a tubular shape. The connection needle 50 extends in an axial direction of the prefilled syringe 28 (X direction). The needle tip of the connection needle 50 is oriented in the X2 direction. In an initial state of the liquid medicine administration device 12, the needle tip of the connection needle 50 is away from the sealing member 36 in an X1 direction.

The needle connection portion 52 connects a proximal end of the connection needle 50 and a proximal end of the puncture needle 46 to each other. The needle connection portion 52 is a flexible tube that causes a lumen of the connection needle 50 and a lumen of the puncture needle 46 to communicate with each other. The cylinder 54 covers the connection needle 50. The cylinder 54 is opened in the X2 direction.

The housing 16 extends in the X direction. The housing 16 includes a first cover member 56 and a second cover member 58. The first cover member 56 includes a bottom wall 60 of the housing 16. A sheet-shaped adhesion member 20 is fixed to a first outer surface 61, which is an outer surface of the bottom wall 60.

The first cover member 56 includes a first peripheral wall 62 protruding in a direction from an outer periphery of the bottom wall 60 toward a ceiling 64 of the housing 16 (Z2 direction). The second cover member 58 includes the ceiling 64 of the housing 16. The second cover member 58 includes a second peripheral wall 66 protruding in a direction from an outer periphery of the ceiling 64 toward the bottom wall 60 of the housing 16 (Z1 direction).

The first peripheral wall 62 and the second peripheral wall 66 form a peripheral wall 68 of the housing 16 by a protruding end of the first peripheral wall 62 and a protruding end of the second peripheral wall 66 engaging with each other. A second outer surface 69, which is an outer surface of the peripheral wall 68, is adjacent to the first outer surface 61.

As illustrated in Fig. 3, in the liquid medicine administration device 12, a first opening 70 and a second opening 72 are formed so as to penetrate the bottom wall 60 of the housing 16 and the adhesion member 20 in the Z direction. The first opening 70 and the second opening 72 are located at one end (end in the X1 direction) of the bottom wall 60. The first opening 70 and the second opening 72 are arranged side by side in a width direction of the housing 16 (Y direction).

The first opening 70 is a circular hole. The first opening 70 is a needle leadout port 73 for causing the puncture needle 46 of the puncture unit 26 to protrude outward from the housing 16 toward the skin 202 of the living body 200. The second opening 72 is a slit extending in the Y direction. The second opening 72 is a hole for inserting a part of the attachment 18 from the outside to the inside of the housing 16.

As illustrated in Figs. 1 and 2, the ceiling 64 of the housing 16 is provided with a window 74 for visually recognizing the liquid medicine in the prefilled syringe 28 located inside the housing 16. As a result, a remaining amount of the liquid medicine in the prefilled syringe 28 can be easily checked. The ceiling 64 of the housing 16 is provided with an operation cover 76. When the operation cover 76 is operated (pressed), the puncture needle 46 protrudes from the needle leadout port 73. An insertion hole 78 for inserting a part of the syringe pump 24 into the housing 16 is provided at an end in the X2 direction of the housing 16.

As illustrated in Fig. 1, in the initial state of the liquid medicine administration device 12, the attachment 18 is attached to the housing 16. As illustrated in Fig. 2, in an attached state of the attachment 18, the attachment 18 holds the puncture unit 26 and the prefilled syringe 28 in a sterile state. The attachment 18 includes an unsealing unit 80, a puncture seal 82, and a connection seal 84.

The unsealing unit 80 is formed of a hard resin material. Examples of a component of the unsealing unit 80 include, but are not limited to, polyacetal. The unsealing unit 80 extends in the Y direction. The unsealing unit 80 is attachable to and detachable from an attachment groove 86 provided in the housing 16. In the initial state of the liquid medicine administration device 12, the unsealing unit 80 is attached to the housing 16 so as to cover a part of the adhesion member 20 from the outside (refer to Fig. 1).

In the attached state of the attachment 18, the puncture seal 82 seals the puncture needle 46 by sealing the needle leadout port 73. The puncture seal 82 is provided in the unsealing unit 80. In the attached state of the attachment 18, the connection seal 84 seals the opening in the X2 direction of the cylinder 54 in a state of being inserted into the second opening 72, thereby sealing the connection needle 50. The connection seal 84 abuts the cylinder 54 in an initial state to seal the connection needle 50.

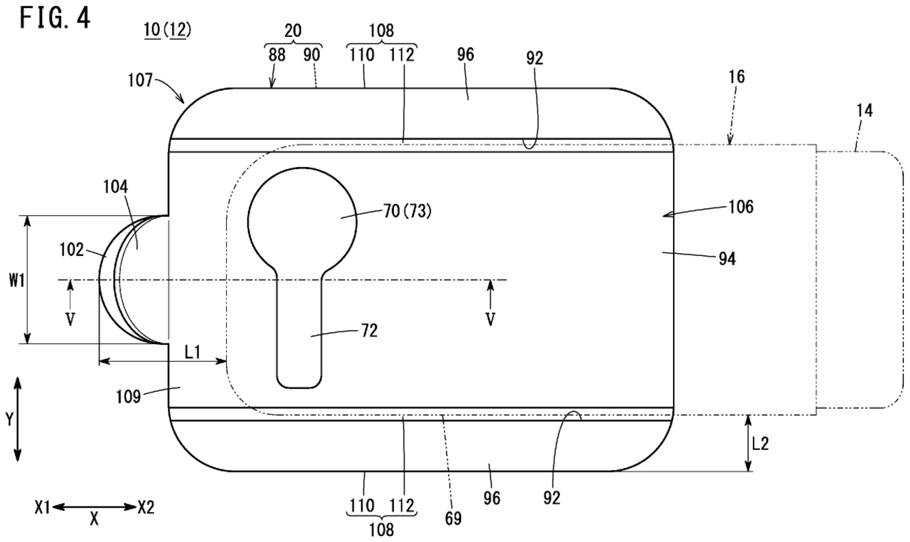

As illustrated in Figs. 4 to 6, the adhesion member 20 is a flexible sheet-shaped member. The adhesion member 20 includes a base sheet 88 and an adhesion sheet 90. The base sheet 88 is a flexible sheet made of resin. Examples of a component of the base sheet 88 include, but are not limited to, polyethylene terephthalate (PET) and the like.

By providing the base sheet 88 with two sheet removed portions 92, the base sheet 88 is divided into a first base 94 and two second bases 96. The sheet removed portion 92 linearly extends over an entire length in an extending direction of the base sheet 88 (X direction). The length in the X direction of the sheet removed portion 92 may be shorter than the length in the X direction of the base sheet 88. The sheet removed portion 92 is only required to be provided at least in a portion deformed (in the present embodiment, curved and deformed) between the attachment 18 and the housing 16 in the attached state of the attachment 18.

A part of the first base 94 is fixed to the first outer surface 61 of the bottom wall 60 of the housing 16. A width in the Y direction of the first base 94 is the same (substantially the same) as a width in the Y direction of the outer surface of the bottom wall 60 of the housing 16. The two second bases 96 are located on both sides in the Y direction with respect to the first base 94. The sheet removed portion 92 is provided between the first base 94 and the second base 96. In other words, the first base 94 is located between the two second bases 96 in the Y direction. The two second bases 96 are not fixed to the housing 16.

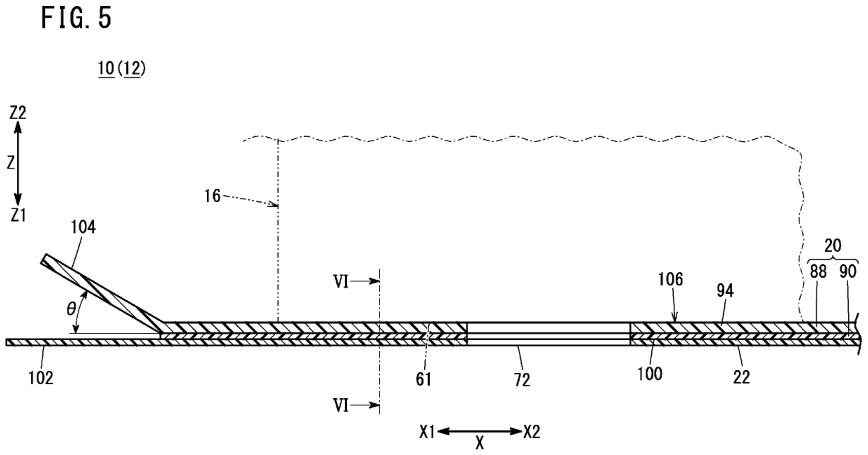

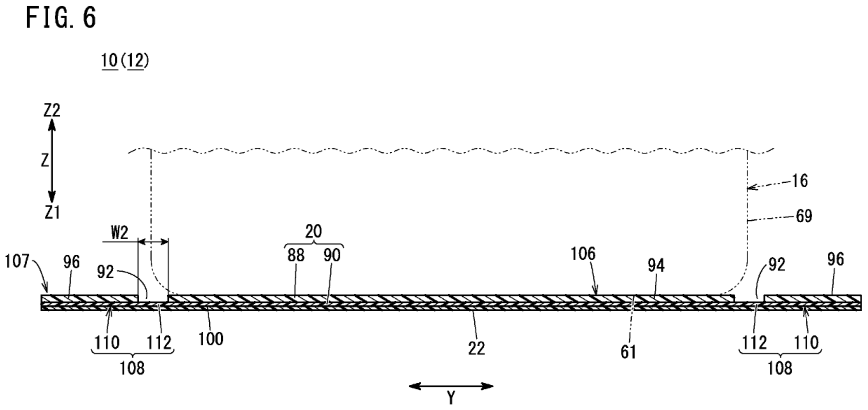

As illustrated in Figs. 5 and 6, the adhesion sheet 90 is fixed to a surface on a side opposite to the housing 16 of the base sheet 88. The adhesion sheet 90 is formed softer than the base sheet 88. In other words, rigidity of the adhesion sheet 90 is lower than that of the base sheet 88. The adhesion sheet 90 includes an adhesion surface 100 facing a side opposite to the base sheet 88 (Z1 direction). The adhesion surface 100 is an adhesive surface to be adhered to the skin 202 of the living body 200. For example, an entire adhesion sheet 90 is formed of a bond (adhesive). Note that, the adhesion sheet 90 may be formed by applying a bond (adhesive) to one surface of a sheet having no adhesive force.

The release sheet 22 is releasably adhered to the adhesion surface 100 in the initial state of the liquid medicine administration device 12. The release sheet 22 is detached from the adhesion surface 100 when the liquid medicine administration device 12 is used. The release sheet 22 is adhered to the entire adhesion surface 100. The release sheet 22 protects the adhesion surface 100. The release sheet 22 is, for example, one continuous sheet of thin paper (release paper).

As illustrated in Figs. 4 and 5, a first tab 102 is provided on an outer periphery of the release sheet 22. The first tab 102 extends outward from the adhesion sheet 90. That is, the first tab 102 is not provided with the adhesion sheet 90. The first tab 102 is provided at an end in the X1 direction of the release sheet 22. The first tab 102 is located at the center in the Y direction of the release sheet 22. The first tab 102 is formed in a semicircular shape.

As illustrated in Fig. 4, a width dimension W1 in the Y direction of the first tab 102 is set to, for example, 10 mm or larger. A length L1 in the X direction from the end in the X1 direction of the housing 16 to an extending end of the first tab 102 is set to 15 mm or longer. In this case, it is easy to pinch the first tab 102 with fingers. A size and a shape of the first tab 102 can be set as appropriate.

As illustrated in Figs. 4 and 5, a second tab 104 is provided on an outer periphery of the base sheet 88. The second tab 104 protrudes in the X1 direction from the end in the X1 direction of the first base 94. A size and a shape of the second tab 104 are the same as those of the first tab 102. The second tab 104 is not provided with the adhesion sheet 90.

As illustrated in Fig. 5, the second tab 104 extends from the base sheet 88 in the X1 direction so as to be inclined toward a side opposite to the release sheet 22 (Z2 direction). The first tab 102 and the second tab 104 are located so as to overlap each other and separated from each other as viewed in the thickness direction of the adhesion sheet 90 (Z direction) (refer to Figs. 4 and 5). An inclination angle θ of the second tab 104 is set to, for example, 30°. The inclination angle θ can be set as appropriate, and may be an angle larger than 30° (for example, 45° or 60°) or an angle smaller than 30° (for example, 20°).

The positions of the first tab 102 and the second tab 104 can be set as appropriate. The first tab 102 may be provided at an end in the Y direction or an end in the X2 direction of the release sheet 22, for example. The second tab 104 may be provided on the second base 96.

As illustrated in Fig. 1, the first tab 102 is preferably provided at a position avoiding a portion folded back by the attachment 18 of the release sheet 22 in the attached state of the attachment 18. Therefore, it is preferable that the first tab 102 is not provided at a corner in the X1 direction of the release sheet 22. Similarly, the second tab 104 is preferably provided at a position avoiding a portion folded back by the attachment 18 of the adhesion member 20 in the attached state of the attachment 18. Therefore, it is preferable that the second tab 104 is not provided at the corner in the X1 direction of the adhesion member 20. The first tab 102 and the second tab 104 may be provided at positions not overlapping each other in the Z direction.

As illustrated in Fig. 4, the adhesion member 20 includes an adhesion body 106 and an overhanging portion 107. The adhesion body 106 is a portion fixed to the outer surface of the bottom wall 60 of the housing 16 out of the adhesion member 20. The adhesion body 106 includes the first base 94 and the adhesion sheet 90.

The overhanging portion 107 is a portion extending from the adhesion body 106 so as to overhang outward from the housing 16 of the adhesion member 20. The overhanging portion 107 extends in the X1 direction and the Y direction with respect to the housing 16. The overhanging portion 107 includes a pair of side overhanging portions 108 and an intermediate overhanging portion 109. The pair of side overhanging portions 108 are located on both sides in the Y direction with respect to the first outer surface 61 of the bottom wall 60 of the housing 16. The side overhanging portion 108 extends over an entire length in the X direction of the adhesion member 20. The intermediate overhanging portion 109 connects portions in the X1 direction with respect to the first outer surface 61 of the pair of side overhanging portions 108.

A length (overhanging length L2) in the Y direction from the second outer surface 69 of the housing 16 to an extending end of the side overhanging portion 108 is preferably 7 mm or longer and 20 mm or shorter, and more preferably 8.5 mm or longer and 20 mm or shorter. When the overhanging length L2 is 7 mm or longer, the overhanging portion 107 is easily pressed with fingers when the adhesion member 20 is adhered to the skin 202 of the living body 200, so that the adhesion member 20 can be excellently adhered to the skin 202 of the living body 200. When the overhanging length L2 is 8.5 mm or longer, the overhanging portion 107 is more easily pressed with fingers when the adhesion member 20 is adhered to the skin 202 of the living body 200. When the overhanging length L2 is 20 mm or shorter, it is possible to reduce pain when the adhesion member 20 is peeled off from the skin 202.

The side overhanging portion 108 is pressed by the attachment 18 in the attached state of the attachment 18 and elastically deformed along the second outer surface 69 of the peripheral wall 68 of the housing 16 (refer to Fig. 1). As illustrated in Figs. 4 and 6, the side overhanging portion 108 includes an overhanging body 110 and an easily deformable portion 112. The overhanging body 110 is formed by stacking the second base 96 and the adhesion sheet 90. That is, the overhanging body 110 includes at least a part of the base sheet 88.

The easily deformable portion 112 is provided in a portion deformed in the attached state of the attachment 18 of the side overhanging portion 108. The easily deformable portion 112 is located in a portion in which the sheet removed portion 92 is provided of the adhesion member 20. That is, the easily deformable portion 112 includes the adhesion sheet 90 but does not include the base sheet 88. Therefore, rigidity of the easily deformable portion 112 is lower than that of the overhanging body 110.

A pair of easily deformable portions 112 are adjacent to the first outer surface 61 of the bottom wall 60 of the housing 16 on both sides in the Y direction. The easily deformable portion 112 is located at a site facing a corner connecting the bottom wall 60 and the first peripheral wall 62 of the housing 16 in the attached state of the attachment 18. In other words, the easily deformable portion 112 is at a root of the side overhanging portion 108. The easily deformable portion 112 extends over an entire length of the adhesion member 20 in the extending direction of the housing 16 (X direction).

The length in the X direction of the easily deformable portion 112 may be shorter than the length in the X direction of the adhesion member 20. The easily deformable portion 112 is only required to be provided at least in a portion deformed (in the present embodiment, curved to be deformed) between the attachment 18 and the housing 16 in the attached state of the attachment 18. As illustrated in Fig. 6, a width dimension W2 in the Y direction of the easily deformable portion 112 (the width dimension of the sheet removed portion 92) can be set as appropriate, but is larger than a thickness dimension of the base sheet 88, for example.

Next, a method of using the liquid medicine administration device 12 will be described.

As illustrated in Fig. 1, in the initial state of the liquid medicine administration device 12, the attachment 18 is attached to the housing 16. Therefore, the side overhanging portion 108 is bent by the attachment 18. In the present embodiment, in the initial state of the liquid medicine administration device 12, the side overhanging portion 108 is deformed so as to face the second outer surface 69 of the peripheral wall 68 of the housing 16.

In a case of using the liquid medicine administration device 12, the user detaches the attachment 18 from the housing 16. As a result, the sealing of the puncture needle 46 by the puncture seal 82 is unsealed, and the sealing of the connection needle 50 by the connection seal 84 is unsealed.

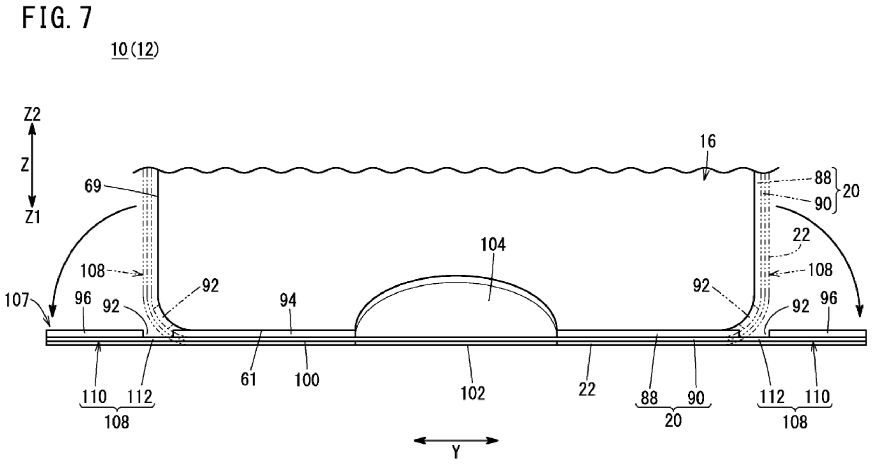

Then, as illustrated in Fig. 7, the deformed (elastically deformed) portion of the side overhanging portion 108 returns to its original shape by an elastic force. In the present embodiment, since the easily deformable portion 112 is provided in the adhesion member 20, the side overhanging portion 108 is prevented from rising in the Z2 direction with respect to the adhesion body 106 in a state in which the attachment 18 is detached from the housing 16. That is, when the attachment 18 is detached from the housing 16, deformation when attaching the attachment 18 hardly remains in the side overhanging portion 108. Therefore, the entire adhesion member 20 extends along an XY plane in a state in which the attachment 18 is detached from the housing 16.

Subsequently, an end in the X2 direction of the drive unit 30 is pushed in the X1 direction with respect to the housing 16. As a result, since the connection needle 50 penetrates the sealing member 36 of the prefilled syringe 28, the liquid medicine in the prefilled syringe 28 can be delivered to the puncture needle 46.

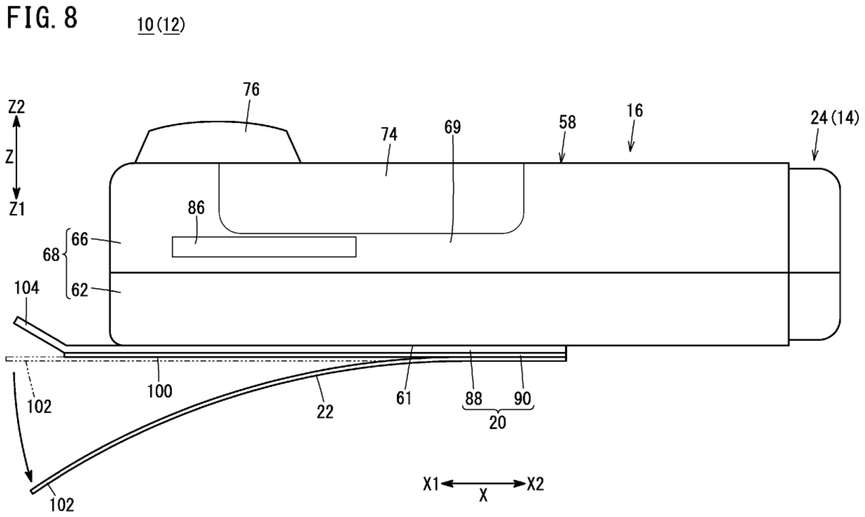

Next, as illustrated in Fig. 8, the release sheet 22 is released from the adhesion sheet 90 in a state in which the first tab 102 is pinched with fingers. As a result, the adhesion surface 100 is exposed to the outside. After the release sheet 22 is released, as illustrated in Fig. 9, the adhesion surface 100 is adhered to the skin 202 of the living body 200. Specifically, the overhanging portion 107 having appropriate rigidity is pressed against the skin 202 with fingers. Thereafter, the operation cover 76 is pressed to puncture the skin 202 with the puncture needle 46, whereby the liquid medicine is administered to the living body 200.

In a case where the administration of the liquid medicine to the living body 200 is finished and the liquid medicine administration device 12 is detached from the skin 202, the second tab 104 is pulled up in the Z2 direction in a state of being pinched with fingers. As a result, the adhesion surface 100 can be smoothly removed from the skin 202.

According to the present embodiment, since the side overhanging portion 108 is deformed along the second outer surface 69 of the housing 16 in the attached state of the attachment 18, the medical device 10 (liquid medicine administration device 12) can be made compact. Since the easily deformable portion 112 is provided in the deformed portion of the side overhanging portion 108, the side overhanging portion 108 easily returns to its original shape when the attachment 18 is detached from the housing 16. That is, it is possible to prevent the deformation from remaining so that the side overhanging portion 108 rises to the side opposite to the direction in which the adhesion surface 100 faces with respect to the adhesion body 106 when the attachment 18 is detached from the housing 16. As a result, the entire adhesion surface 100 can be held in a planar shape when the attachment 18 is detached from the housing 16. Since the overhanging portion 107 includes the overhanging body 110 having higher rigidity than that of the easily deformable portion 112, the overhanging portion 107 can be easily pressed with fingers as compared with a case where the entire overhanging portion 107 is formed of the easily deformable portion 112. Therefore, the adhesion member 20 can be stably adhered to the skin 202 of the living body 200.

The adhesion member 20 includes the adhesion sheet 90 including the adhesion surface 100 and the base sheet 88 provided on a surface on a side opposite to the adhesion surface 100 of the adhesion sheet 90 to support the adhesion sheet 90. According to such a configuration, it is easy to set the adhesion member 20 to have appropriate rigidity by the base sheet 88.

The overhanging body 110 includes at least a part of the base sheet 88, and the easily deformable portion 112 does not include the base sheet 88. According to such a configuration, the easily deformable portion 112 can be easily provided in the side overhanging portion 108.

The housing 16 extends in one direction. The side overhanging portion 108 extends outward in the width direction intersecting the extending direction of the housing 16 from the adhesion body 106. The easily deformable portion 112 extends over an entire length of the adhesion member 20 in the extending direction of the housing 16. According to such a configuration, a dimension of the medical device 10 in the width direction of the housing 16 can be made compact in a state in which the attachment 18 is attached to the housing 16.

The release sheet 22 is releasably provided on the adhesion surface 100. The first tab 102 is provided on the outer peripheral portion of the release sheet 22, and the adhesion member 20 is not provided on the first tab 102. According to such a configuration, the release sheet 22 can be released from the adhesion surface 100 in a state in which the first tab 102 is pinched.

The second tab 104 is provided on the outer periphery of adhesion member 20. The second tab 104 is not provided with the adhesion surface 100. According to such a configuration, the adhesion surface 100 can be released from the skin 202 in a state in which the second tab 104 is pinched.

The first tab 102 and the second tab 104 are located so as to overlap each other and separated from each other as viewed in the thickness direction of the adhesion member 20. According to such a configuration, since the first tab 102 and the second tab 104 can be simultaneously processed, the number of manufacturing steps of the medical device 10 can be reduced. The configuration of the medical device 10 is simplified. Furthermore, since the first tab 102 and the second tab 104 are separated from each other, only the first tab 102 can be easily pinched when the release sheet 22 is released from the adhesion surface 100.

The release sheet 22 is one continuous sheet. According to such a configuration, a releasing operation of the release sheet 22 can be efficiently performed.

Inside the housing 16, the prefilled syringe 28 and the puncture needle 46 for administering the liquid medicine accommodated in the prefilled syringe 28 to the living body 200 are provided. In the housing 16, the needle leadout port 73 for exposing the needle tip of the puncture needle 46 is formed, and the attachment 18 seals the needle leadout port 73 in a state of being attached to the housing 16. According to such a configuration, it is possible to obtain the medical device 10 (liquid medicine administration device 12) for administering the liquid medicine to the living body 200.

### (First Modification)

Next, an adhesion member 20a according to a first modification will be described. In the present modification, the configuration the same as that of the above-described adhesion member 20 of the adhesion member 20a is denoted by the same reference numeral, and a detailed description thereof will be omitted. This is similar for the adhesion member 20b according to a second modification to be described later.

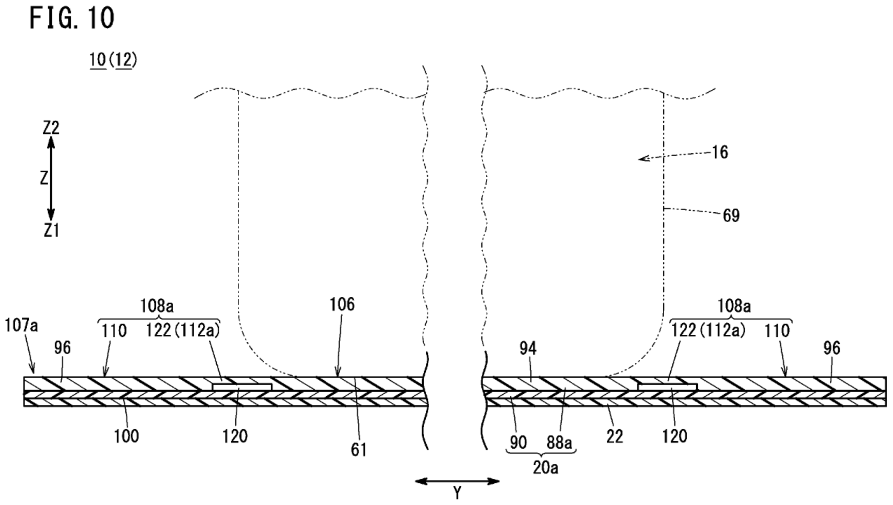

As illustrated in Fig. 10, the adhesion member 20a includes a base sheet 88a and the adhesion sheet 90. The base sheet 88a is provided with a groove 120 instead of the sheet removed portion 92. The groove 120 is formed on a surface facing a side opposite to the housing 16 (Z1 direction) of the base sheet 88a.

The groove 120 extends over an entire length in the X direction of the base sheet 88a. The length in the X direction of the groove 120 may be shorter than the length in the X direction of the base sheet 88a. The groove 120 is only required to be provided at least in a portion deformed (in the present embodiment, curved to be deformed) between the attachment 18 and the housing 16 in the attached state of the attachment 18. The width dimension of the groove 120 is similar to the width dimension of the sheet removed portion 92 described above. The base sheet 88a includes the first base 94, a pair of second bases 96, and a pair of base connection portions 122. The base connection portion 122 connects the first base 94 and the second base 96 to each other. The base connection portion 122 forms a bottom surface of the groove 120.

The adhesion member 20a includes the adhesion body 106 and an overhanging portion 107a. A side overhanging portion 108a of the overhanging portion 107a includes the overhanging body 110 and an easily deformable portion 112a. Each of the adhesion body 106 and the easily deformable portion 112a includes at least a part of the base sheet 88a. The base sheet 88a includes the base connection portion 122 (first portion) forming the easily deformable portion 112a and the second base 96 (second portion) forming the overhanging body 110. The base connection portion 122 is thinner than the second base 96.

In the present modification, the similar effect is obtained for the configuration similar to that of the above-described adhesion member 20a.

Each of the overhanging body 110 and the easily deformable portion 112a includes at least a part of the base sheet 88a. The base sheet 88a includes the base connection portion 122 forming the easily deformable portion 112a and the second base 96 forming the overhanging body 110. The base connection portion 122 is thinner than the second base 96. According to such a configuration, the easily deformable portion 112a can be easily provided in the side overhanging portion 108a.

The base sheet 88a is provided with the groove 120, and the base connection portion 122 is formed of the groove 120. According to such a configuration, the rigidity of the easily deformable portion 112a can be easily reduced.

The groove 120 is formed on a surface facing the adhesion sheet 90 of the base sheet 88a. According to such a configuration, when the attachment 18 is attached to the housing 16, the side overhanging portion 108a can be easily bent to the side opposite to the adhesion surface 100. Therefore, the attachment 18 can be easily attached to the housing 16.

### (Second Modification)

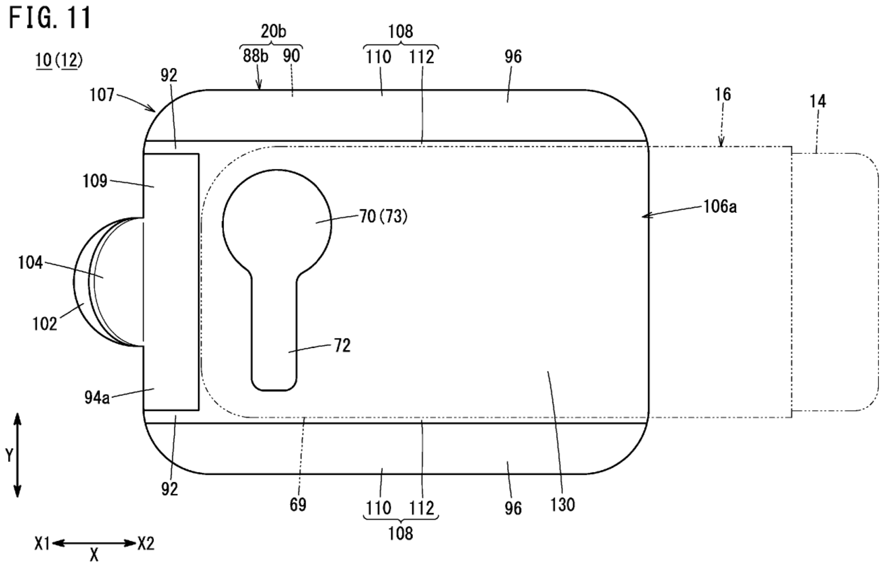

Next, an adhesion member 20b according to a second modification will be described. As illustrated in Fig. 11, the adhesion member 20b includes a base sheet 88b and the adhesion sheet 90. The base sheet 88b includes a first base 94a and a pair of second bases 96.

A central sheet removed portion 130 that connects a pair of sheet removed portions 92 to each other is formed on the first base 94a. The central sheet removed portion 130 is located at a position corresponding to the first outer surface 61 of the bottom wall 60 of the housing 16. In the adhesion member 20b, the base sheet 88b is not present at a position facing the first outer surface 61 of the bottom wall 60 of the housing 16. Therefore, the first outer surface 61 of the bottom wall 60 of the housing 16 is fixed to the adhesion sheet 90.

The pair of sheet removed portions 92 extend in the X1 direction from the central sheet removed portion 130. The adhesion member 20b includes an adhesion body 106a and the overhanging portion 107. The adhesion body 106a does not include the base sheet 88b but includes the adhesion sheet 90.

In the present modification, the similar effect is obtained for the configuration similar to that of the above-described adhesion member 20a.

The adhesion body 106a does not include the base sheet 88b. According to such a configuration, the configuration of the adhesion member 20b can be further simplified.

In the present modification, the adhesion member 20b may be provided with the groove 120 described above instead of the sheet removed portion 92.

### (Third Modification)

Next, an adhesion member 20c according to a third modification will be described. In the present modification, the configuration the same as that of the adhesion member 20b according to the above-described second modification of the adhesion member 20c is denoted by the same reference numeral, and a detailed description thereof will be omitted.

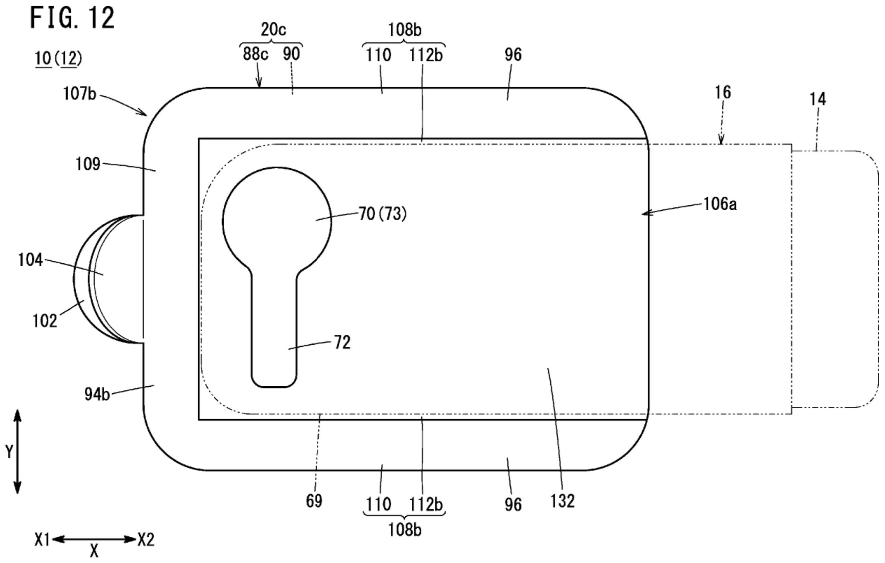

As illustrated in Fig. 12, the adhesion member 20c includes a base sheet 88c and the adhesion sheet 90. The base sheet 88c is provided with a sheet removed portion 132. The sheet removed portion 132 has a size and a shape in which the first outer surface 61 of the bottom wall 60 of the housing 16 can be disposed. The width dimension in the Y direction of the sheet removed portion 132 is larger than the width dimension in the Y direction of the housing 16. An end in the X1 direction of the sheet removed portion 132 is located in the X2 direction with respect to an end in the X1 direction of the adhesion member 20c.

The base sheet 88c includes a first base 94b and two second bases 96. The first base 94b is located in the X1 direction with respect to the sheet removed portion 132. The first base 94b extends so as to connect the pair of second bases 96 to each other. That is, the base sheet 88c is not divided into a plurality of sheets and is one continuous sheet.

The adhesion member 20c includes the adhesion body 106a and an overhanging portion 107b. The overhanging portion 107b includes a pair of side overhanging portions 108b and the intermediate overhanging portion 109. The side overhanging portion 108b is provided with the overhanging body 110 and an easily deformable portion 112b. The easily deformable portion 112b does not include the base sheet 88c but includes the adhesion sheet 90. A length in the X direction of the easily deformable portion 112b is shorter than the length in the X direction of the side overhanging portion 108b. The easily deformable portion 112b is not provided at an end in the X1 direction of the side overhanging portion 108b.

In the present modification, the similar effect is obtained for the configuration similar to that of the above-described adhesion member 20b.

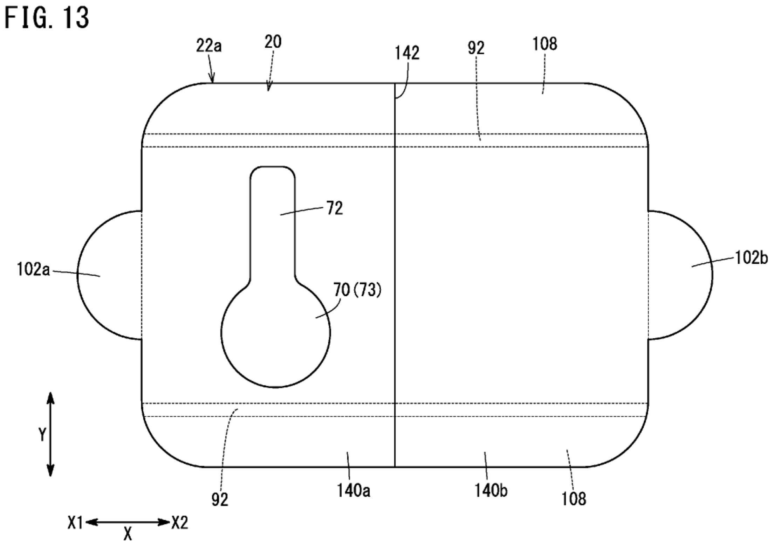

The liquid medicine administration device 12 may include a release sheet 22a illustrated in Fig. 13 instead of the release sheet 22 described above. Fig. 13 is a plan view of the release sheet 22a as viewed from a side opposite to the housing 16 (Z1 direction). As illustrated in Fig. 13, the release sheet 22a is divided into two divided sheets 140a and 140b. A division line 142 of the release sheet 22a is provided at a position away from the first opening 70 and the second opening 72. The division line 142 is not connected to outer peripheries of the first opening 70 and the second opening 72. The division line 142 is provided at a position shifted in the X2 direction from the first opening 70 and the second opening 72. The division line 142 extends linearly in the Y direction. The divided sheet 140a is adjacent to the divided sheet 140b in the X1 direction.

The two divided sheets 140a and 140b have the same outer size and shape. Note that, the two divided sheets 140a and 140b may have different outer sizes and shapes.

The two divided sheets 140a and 140b are provided with the first tabs 102a and 102b, respectively. The first tab 102a is provided at an end in the X1 direction of the divided sheet 140a. The first tab 102b is provided at an end in the X2 direction of the divided sheet 140b.

The first opening 70 and the second opening 72 penetrating the adhesion member 20 and the release sheet 22a in the thickness direction of the adhesion member 20 are formed in the adhesion member 20 and the release sheet 22a. The release sheet 22a is divided into the plurality of divided sheets 140a and 140b by the division line 142. The division line 142 is provided at a position away from the first opening 70 and the second opening 72 so as not to be connected to the outer peripheries of the first opening 70 and the second opening 72. According to such a configuration, it is possible to suppress the release sheet 22a from being broken when the release sheet 22a is released from the adhesion surface 100.

The release sheet 22a can be provided on the adhesion surface 100 of the adhesion members 20a to 20c described above. The division line 142 may extend in a curved shape or may extend in the X direction. The release sheet may be divided into three or more divided sheets by division lines.

Note that, the present invention is not limited to the above disclosure, and various configurations can be adopted without departing from the gist of the present invention.

## Claims

1. A medical device comprising: a housing; and a flexible sheet-shaped adhesion member that is provided in the housing and comprises an adhesion surface capable of being adhered to a skin of a living body,
the medical device comprising an attachment that is detachably attached to the housing,
wherein
the adhesion member comprises:
an adhesion body that is fixed to a first outer surface of the housing, and
an overhanging portion that extends from the adhesion body so as to overhang outward from the housing, and is pushed by the attachment in an attached state in which the attachment is attached to the housing and deforms along a second outer surface adjacent to the first outer surface of the housing,
the overhanging portion comprises:
an overhanging body, and
an easily deformable portion having lower rigidity than rigidity of the overhanging body, and
the easily deformable portion is provided at least a portion deformed between the attachment and the housing in the attached state of the overhanging portion.

2. The medical device according to claim 1, wherein
the adhesion member comprises:
an adhesion sheet comprising the adhesion surface, and
a base sheet provided on a surface on a side opposite to the adhesion surface of the adhesion sheet to support the adhesion sheet.

3. The medical device according to claim 2, wherein
the overhanging body comprises at least a part of the base sheet, and
the easily deformable portion does not comprise the base sheet.

4. The medical device according to claim 2, wherein
each of the overhanging body and the easily deformable portion comprises at least a part of the base sheet,
the base sheet comprises:
a first portion that forms the easily deformable portion, and
a second portion that forms the overhanging body, and
the first portion is thinner than the second portion.

5. The medical device according to claim 4, wherein
the base sheet is provided with a groove, and
the first portion is formed of the groove.

6. The medical device according to claim 5, wherein
the groove is formed on a surface facing the adhesion sheet of the base sheet.

7. The medical device according to claim 2, wherein
the adhesion body does not comprise the base sheet.

8. The medical device according to claim 1, wherein
the housing extends in one direction,
the overhanging portion extends outward in a width direction intersecting an extending direction of the housing from the adhesion body, and
the easily deformable portion extends over an entire length of the adhesion member in the extending direction of the housing.

9. The medical device according to claim 1, wherein
a release sheet is provided on the adhesion surface so as to be releasable,
a tab is provided on an outer periphery of the release sheet, and
the tab is not provided with the adhesion member.

10. The medical device according to claim 9, wherein
the tab is a first tab,
a second tab is provided on an outer periphery of the adhesion member, and
the second tab is not provided with the adhesion surface.

11. The medical device according to claim 10, wherein
the first tab and the second tab are located so as to overlap each other and separated from each other as viewed in a thickness direction of the adhesion member.

12. The medical device according to claim 1, wherein
a release sheet is releasably provided on the adhesion surface, and
the release sheet is one continuous sheet.

13. The medical device according to claim 1, wherein
a release sheet is releasably provided on the adhesion surface,
an opening penetrating the adhesion member and the release sheet is formed in the adhesion member and the release sheet in a thickness direction of the adhesion member,
the release sheet is divided into a plurality of divided sheets by a division line, and
the division line is provided at a position away from the opening so as not to be connected to an outer periphery of the opening.

14. The medical device according to any one of claims 1 to 13, wherein
a prefilled syringe and a puncture needle for administering a liquid medicine accommodated in the prefilled syringe to a living body are provided inside the housing,
a needle leadout port for exposing a needle tip of the puncture needle is formed in the housing, and
the attachment seals the needle leadout port in a state of being attached to the housing.
